# EUROPEAN PATENT APPLICATION

(11) **EP 4 664 475 A1**
(43) Date of publication of application: **17.12.2025**
(21) Application number: 24182143.8
(22) Date of filing: 14.06.2024
(51) Int. Cl.: G16H 40/40, G16H 40/67, A61N 1/372, G06Q 10/20, G06Q 30/06

(54) **SYSTEM AND METHOD FOR MANAGING OPERATION OF A MEDICAL DEVICE**

(71) Applicant: BIOTRONIK SE & Co. KG, 12359 Berlin (DE)
(72) Inventor: TAFF, Brian M., Portland, 97217 (US)
(74) Representative: Biotronik Corporate Services SE

(57) **Abstract**

A system for managing operation of a medical device (1, 1', 1") comprises a medical device (1, 1', 1") configured to carry out therapeutic and/or diagnostic functions in or on a patient (P), wherein the medical device (1, 1', 1") comprises a configurable firmware for controlling operation of the medical device (1, 1', 1") for carrying out said therapeutic and/or diagnostic functions. A remote management system (3) is configured to effect an adaption of a configuration of the configurable firmware of the medical device (1, 1', 1") based on information relating to a subscription by which the patient (P) has subscribed to a medical service provided by operation of the medical device (1, 1', 1").

## Description

The instant invention generally relates to a system for managing operation of a medical device and to a method for operating such a system.

A system of this kind comprises a medical device configured to carry out therapeutic and/or diagnostic functions in or on a patient. The medical device generally comprises a configurable firmware for controlling operation of the medical device for carrying out the therapeutic and/or diagnostic functions.

In addition, the system comprises generally a remote management device positioned remotely from the patient.

The medical device may for example be an implantable medical device. An implantable medical device may for example be a pacemaker, an implantable cardioverter defibrillator, a sensor device such as a bio-sensor, or a monitoring device. The implantable medical device in other embodiments may also be a neuro-stimulation device.

In other embodiments, the medical device may be a wearable device or a point-of-care device.

Typically, for example implantable medical devices such as cardiac stimulation devices or neuro-stimulation devices are provided as dedicated devices for dedicated purposes, i.e., for providing dedicated functions within the patient, for example to provide for a specific cardiac pacing and/or detection function. If, subsequent to implanting the implantable medical device in a patient, a function shall be adapted or other functions shall be added, this generally requires to remove the implantable medical device from the patient and to replace the implantable medical device by another device. This is cumbersome and potentially poses a significant risk for the patient.

It is an object to provide a system for managing operation of a medical device and a method for operating such a system which allow, in an easy and efficient way, to provide variable functions in or on a patient, while having in mind a potential for a commercial exploitation by a device manufacturer.

In one aspect, a system for managing operation of a medical device comprises a medical device configured to carry out therapeutic and/or diagnostic functions in or on a patient, wherein the medical device comprises a configurable firmware for controlling operation of the medical device for carrying out said therapeutic and/or diagnostic functions, and a remote management system, wherein the remote management system is configured to effect an adaption of a configuration of the configurable firmware of the medical device based on information relating to a subscription by which the patient has subscribed to a medical service provided by operation of the medical device.

The system comprises, in one aspect, a medical device and a remote management device. The medical device is configured to carry out a therapeutic and/or diagnostic function in or on a patient and for this comprises a configurable firmware. By means of the firmware, operation of the medical device is controlled, the firmware being programmed such that the medical device may carry out its prescribed therapeutic and/or diagnostic function. The remote management system, in turn, is remote from the medical device and, directly or indirectly, is in communication connection with the medical device such that data directly or indirectly may be communicated in between the medical device and the remote management device.

Herein, the remote management device is configured to effect an adaption of a configuration of the configurable firmware of the medical device based on information relating to a subscription by which the patient has subscribed to a medical service provided by operation of the medical device. This allows to operate the medical device according to a subscription model. The patient may subscribe to a particular subscription, and based on the subscription the medical device is operated in order to provide, by operation of the medical device, the medical service to which the patient has subscribed.

Because the remote management system is enabled to effect an adaption of a configuration of the configurable firmware of the medical device, the remote management system may assess information relating to a subscription of a patient and, based on the assessment, may adapt a configuration of the firmware. In particular, the remote management system may check which subscription the patient has subscribed to, and in accordance with the subscription of the patient the remote management system adapts the configuration of the firmware such that the medical device is enabled to offer such services to the patient which are included in the subscription.

In addition, the remote management system may check whether the patient has fulfilled his/her obligations under the subscription, for example whether he/her has made the required payments under the subscription, in order to adapt the configuration of the firmware based on the check. If it is found that the patient has not acted according to his obligations, the configuration of the firmware may be suitably adapted in order to for example reduce a service provided by the medical device.

By means of the remote management system, hence, a tiered management of the medical device becomes possible. For this, the remote management system checks information relating to a subscription of a patient and manages the medical device accordingly by suitably causing an adaption of a configuration of the configurable firmware of the medical device in order to offer medical services by the medical device according to the subscription and according to information relating to the subscription, for example relating to payment information relating to a subscription.

In one embodiment, the medical device is an implantable medical device configured for implantation in a patient.

For example, the implantable medical device may be an implantable cardiac stimulation device, such as an implantable pulse generator (IPG), an implantable cardioverter defibrillator (ICD) or a cardiac resynchronization therapy (CRT-D or CRT-P) device.

In other embodiments, the implantable medical device may be an implantable cardiac monitoring device, such as a bio monitor configured to sense and record cardiac signals, in particular electrocardiogram signals.

In yet other embodiments, the implantable medical device may be an implantable neuro-stimulation device. In particular, by means of a neuro-stimulation device a pain management may be provided. For example, a neuro-stimulation device may provide for a spinal cord stimulation.

In one embodiment, the medical device is a wearable medical device configured to be worn by a patient. In a worn state, the wearable medical device for example is in close proximity to the patient's skin. A wearable medical device of this type may for example be a wristwatch-type device or the like for monitoring body functions, such as the heart rat, blood oxygen, sleep quality and the like.

In one embodiment, the medical device is a medical point-of-care device configured to operate externally to the patient. Point-of-care devices generally are configured to provide diagnostic functions, in particular in-vitro diagnostics near or at the site of a patient. A point-of-care diagnostic device may for example be a glucose monitoring device.

In one embodiment, the remote management system is configured to communicate with a patient device (separate from the medical device) resting outside of the patient. The patient device, for example a smart phone, a tablet computer or a dedicated communication and monitoring device in the context of a home monitoring system, rests at the site of the patient and is configured to communicate with the medical device. The patient device in particular may provide a link in between the medical device, which for example is implanted in the patient, and the remote management device, which may be located in a public environment and may for example be linked to the patient device via a public communication network, such as the Internet. The patient device may for example be configured to communicate with the medical device by means of a close-range communication technology, for example according to, magnetically-inductive wand-based telemetry, wandless Bluetooth, Bluetooth Low Energy (BLE) or Zigbee strategies.

In one embodiment, the firmware is programmed to include a complete set of features for carrying out therapeutic and/or diagnostic functions, wherein the firmware is configurable to enable a subset of features of the complete set of features and disabling features of the complete set of features not included in the subset based on the information relating to a subscription by which the patient has subscribed to a medical service provided by operation of the medical device.

This assumes that the firmware is programmed to include a complete set of features, that is a complete variety of capabilities which the medical device - dependent on its configuration - may offer. The medical device, for example an implantable medical device, hence is implemented with a firmware which is programmed to include a large set of features, relating for example to different functions to be carried out by the medical device for providing a medical service. Although the firmware includes the complete set of features, not all features are necessarily enabled and available to a user. Rather, the remote management system, by configuring the firmware, may enable a subset of the available features based on subscription information. Hence, the remote management system adapts the configuration of the firmware such that only those features are enabled which are included in the subscription of the patient. Other features are disabled, such that they are not accessible and are not offered to the patient.

For example, if a patient has subscribed only to a basic subscription, only a basic set of features may be enabled and may be carried out by the medical device, for example those features relating to a minimum requirement of the medical device for carrying out its basic, essential function. If, in contrast, the patient has described to a higher subscription, an increased set of features may be enabled and may be carried out by the medical device. By means of the increased set of features, additional functions may be available to the patient.

This allows for example that, if the patient decides at a later time to change his subscription, the configuration of the firmware may be adapted, without having to replace the medical device.

In the context of a pain management device, for example an implantable neuro-stimulation device, features which may be selectively enabled or disabled based on a subscription of a patient may relate to pain management options, for example to enable a greater or a reduced control over dynamics of a device response. Such pain management devices, for example neuro-stimulation devices, may in particular provide for a paresthesia-free pain management.

In the context of diagnostic functions of a medical device, features which may be selectively enabled or disabled based on a subscription of a patient may for example relate to a presentation of information to a patient and to a level of recording of diagnostic data. For example, based on the enabling or disabling of features information may be presented to a user with a higher or lower degree of information detail. In addition, information may be more frequently or less frequently updated. Further in addition, a higher-level feature may provide for a graphical display of information to a user, whereas a lower-level feature may provide only for a literal (in plain words) presentation of information to a user. Further in addition, a higher-level feature may provide for a recording of diagnostic data, for example relating to electrocardiogram data, with a higher resolution than for a lower-level feature. In addition, an atrial fibrillation detection function may be enabled or disabled based on a subscription. Further in addition, features relating to the monitoring for example of a patient's activity may be enabled or disabled based on a subscription.

In one embodiment, the remote management system is configured, for effecting the adaption of the configuration, to check a subscription level of the patient and/or to check whether the patient has fulfilled the patient's obligations associated with a subscription level.

In one embodiment, the remote management system is configured to adapt the configuration of the firmware to enable a first set of features for providing a medical service in case the patient has subscribed to a first subscription level and to adapt the configuration of the firmware to enable a second set of features for providing a medical service in case the patient has subscribed to a second subscription level.

Different subscription levels may be defined, such as a basic subscription level, a medium subscription level and a premium subscription level. The patient may subscribe to any of the subscription levels, wherein based on the subscription level the patient has subscribed to the configuration of the firmware is adapted such that such features are enabled which correspond to the subscription level the patient has subscribed to.

In addition, the remote management system may check, for example based on financial records available in relation to the patient, whether the patient has fulfilled the obligations under the subscription level, in particular whether the required payments under the subscription level have been made. If this is not the case, certain features may be disabled such that the patient no longer is offered such features which would be available under the subscription level that the patient has subscribed to.

Generally, if it is found by assessment at the remote management system that certain obligations under a subscription level have not been fulfilled by the patient, features may be disabled such that the disabled features no longer are available to the patient. Herein, for example vital features are not disabled such that a minimum functionality of the medical device is still available to the patient.

In one embodiment, the (remote management and/or medical device) system is configured to unlock or block specific features, if the patient's lifestyle behaviors does not match the requirements or instructions. As an example, if the patient does not "exercise enough" maybe he does not get the pretty or desired graphics (in his app or user interface). This approach may be seen as a means to drive patients toward behaviors that improve the niceties of the system.

In one embodiment, the remote management system is configured, by effecting the adaption of the configuration of the configurable firmware of the medical device, to expand or limit at least one feature for providing a medical service.

A feature may be expanded by enabling a predefined function to be carried out by the medical device or by increasing a capability of a predefined function to be carried out by the medical device. For example, a certain function may be enabled. In another example, a certain function may have been enabled before, but for example a limit may be expanded such that the patient is offered an enhanced functionality, for example for more frequently updating diagnostic information or for expanding capabilities of a control of a pain management.

In contrast, a feature may be limited by disabling a predefined function such that it is not carried out by the medical device or by decreasing a capability of a predefined function to be carried out by the medical device. A function hence may be disabled such that it no longer is carried out by the medical device. In another example, a function may be decreased in its capability, for example by decreasing a limit such that a reduced service capability is offered, for example for less frequently updating diagnostic information or for reducing capabilities of a control of a pain management.

In one embodiment, the medical device is configured to cause an adaption of the configuration of the firmware based on a predefined timeout period following a prior communication with the remote management system. In particular, the medical device may be configured to cause an adaption of the configuration of the firmware to limit at least one feature for providing a medical service in case the predefined timeout period following the prior communication with the remote management system is exceeded without another communication with a remote management system being established within the timeout period. In order to offer services to a patient under a certain subscription, the medical device may be required to regularly check-in with the remote management system. In particular, by requiring the medical device to regularly check-in with the remote management system, it shall be avoided that a patient terminates a subscription and still receives services by operation of the medical device under the prior subscription. By configuring the medical device to check a timeout period it may be ensured that certain features under a subscription are offered to the patient only if a communication is regularly established between the medical device and the remote management system. If the timeout period following a prior communication is exceeded without another communication between the remote management system and medical device having taken place, the medical device may adapt the configuration of the firmware such that certain features are disabled or modified.

In another aspect, a method for operating a system for managing operation of a medical device comprises: providing a medical device configured to carry out therapeutic and/or diagnostic functions in or on a patient, wherein the medical device comprises a configurable firmware for controlling operation of the medical device for carrying out said therapeutic and/or diagnostic functions; providing a remote management system; and effecting, by remote management system, an adaption of a configuration of the configurable firmware of the medical device based on information relating to a subscription by which the patient has subscribed to a medical service provided by operation of the medical device.

The advantages and advantageous embodiments described above for the system equally apply also to the method such that it shall be referred to the above.

The various features and advantages of the present invention may be more readily understood with reference to the following detailed description and the embodiments shown in the drawings. Herein,
- Fig. 1: shows a schematic drawing of a system comprising an implantable medical device;
- Fig. 2: shows a schematic drawing of a system comprising another type of a medical device; and
- Fig. 3: shows a schematic drawing of a medical device communicating with a remote management system.

Subsequently, embodiments of the invention shall be described in detail with reference to the drawings. In the drawings, like reference numerals designate like structural elements.

It is to be noted that the embodiments are not limiting for the invention, but merely represent illustrative examples.

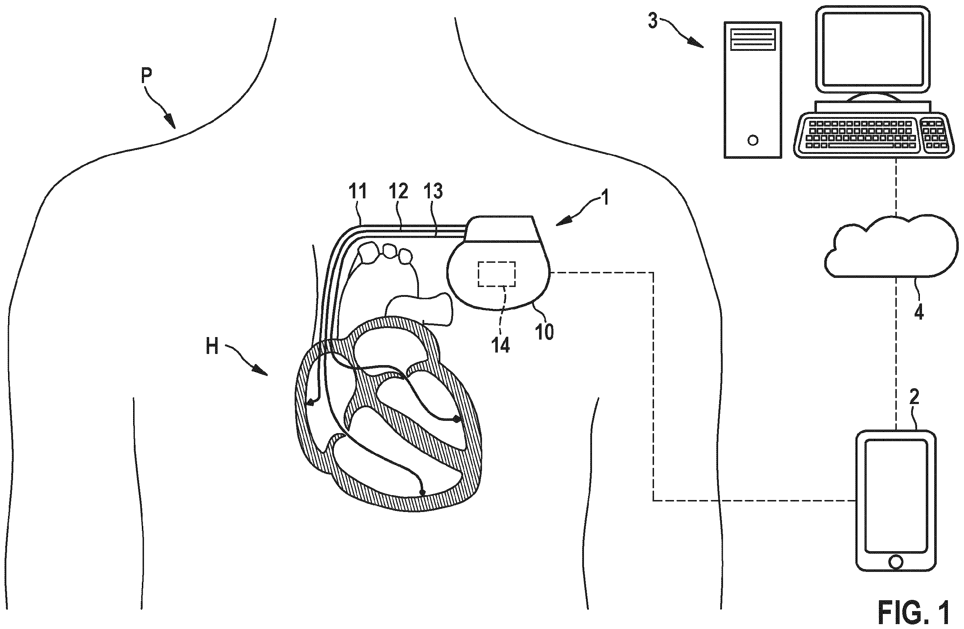

Referring to Fig. 1, in one embodiment a system comprises a medical device formed by an implantable medical device 1 implanted in a patient for serving a therapeutic and/or diagnostic function.

The implantable medical device 1, in the shown embodiment, comprises a generator device 10 and an arrangement of electrode leads 11, 12, 13 extending from the generator device 10. The generator device 10 may for example be implanted subcutaneously into a patient P, the electrode leads 11, 12, 13 reaching into the patient's heart H for monitoring cardiac activity of the patient's heart H. The generator device 10 comprises a processing circuitry 14 encapsulated in a housing of the generator device 10 together with an electrochemical battery for supplying electrical energy for operation of the implantable medical device 1.

An implantable medical device 1 may generally be an active implantable medical device, that is an implantable medical device comprising a battery for performing a therapeutic and/or a diagnostic function (in short AIMD). An implantable medical device 1 may be a stimulation device, such as a cardiac stimulation device or a neuro-stimulation device. In other embodiments, an implantable medical device 1 may be a monitoring device or a sensor device for monitoring a physiological state within the patient P.

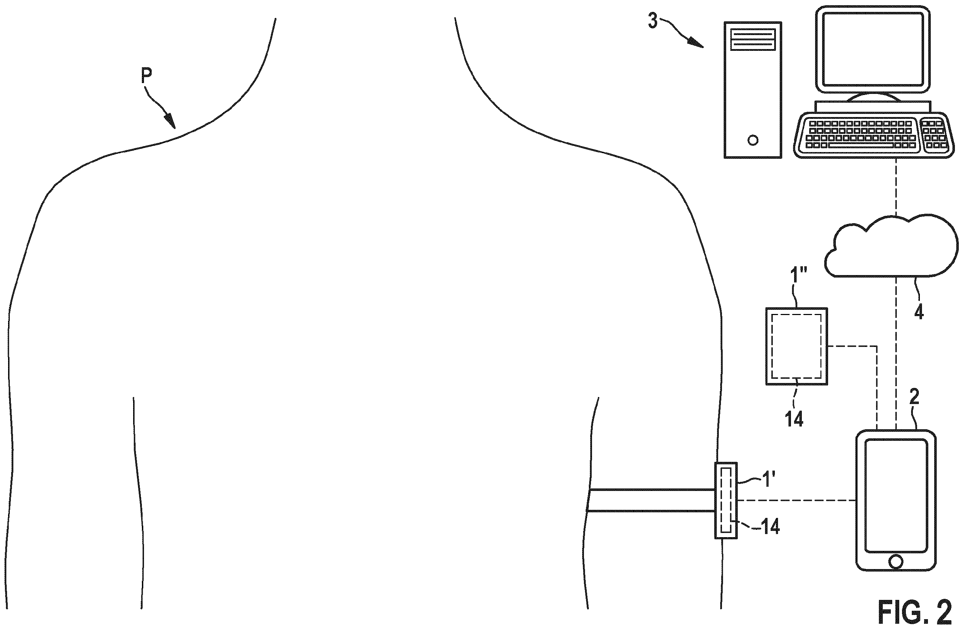

Referring now to Fig. 2, in other examples a medical device may be a wearable device 1' or a point-of-care device 1". A wearable device 1' may for example be a wristwatch-type device and may rest on a skin of the patient P in order to for example sense signals on the skin surface. A point-of-care device 1" may for example be a glucose monitoring device and may rest at the site of a patient P in order to perform in-vitro diagnostics at the site of the patient P.

In any of the examples of Fig. 1 and Fig. 2, the system in addition comprises a patient device 2 external to the patient P and being configured to communicate with the medical device 1, 1', 1". The patient device 2 may be in connection, via a public communication network 4, with a remote management system 3.

The patient device 2 may be an off-the-shelf smart phone or a tablet computer. The patient device 2 may also be a dedicated monitoring device in the context of a home monitoring system.

The patient device 2 may for example have a display in order to display data relating to operation of the medical device 1, 1', 1", for example diagnostic data, such as electrocardiogram signal data for example obtained by an implantable medical device 1 or a wearable device 1'.

The medical device 1, 1', 1" comprises processing circuitry 14 on which a firmware is implemented to control operation of the medical device 1, 1', 1". By means of the firmware certain functions are implemented, for example diagnostic or therapeutic functions, for example for sensing signals such as cardiac electrocardiogram signals or for outputting stimulation signals, for example pacemaker signals or neuro-stimulation signals, for example in the context of a pain management.

Herein, the firmware implemented on the processing circuitry 14 of the respective medical device 1, 1', 1", in one embodiment, is programmed to include a complete set of features for carrying out a therapeutic and/or diagnostic function. The firmware however is configurable to enable (only) a subset of the complete set of features and to disable features which are not included in the subset. Hence, the medical device 1, 1', 1" is programmed such that it includes a set of available features, which however may be selectively enabled or disabled.

The enabling of the disabling of features herein takes place under the control of the remote management system 3, which by communication via the patient device 2 effects an adaption of a configuration of the firmware of the medical device 1, 1', 1" for enabling, disabling or modifying certain features in accordance with an assessment of information relating to a subscription of the patient P.

In one embodiment, services by operating the medical device 1, 1', 1" may be offered to the patient P under a subscription model. The patient P may have subscribed to a certain subscription, and under the subscription certain services shall be offered to the patient P by operating the medical device 1, 1', 1". Herein, generally the higher the subscription level of the patient P, the more services may be offered to the patient P. In turn, the lower the subscription level, the fewer services are offered to the patient P.

For example, under a basic subscription, only basic services may be offered, for example only those services relating to a vital function of the medical device 1, 1', 1". In contrast, under an enhanced subscription, enhanced services may be offered to the patient P, for example services relating to an increased comfort of the patient P or services offering an improved feedback to the patient P, for example an enhanced presentation of diagnostic information or a general activity monitoring of the patient, which is not vital, but may improve the comfort of the patient P.

The remote management system 3, for example a remote server device, for example located at or operated by a device manufacturer, is configured to effect an adaption of the configuration of the firmware of the medical device 1, 1', 1" remotely according to an assessment of information relating to a subscription of the patient P. For example, it may be assessed what subscription level the patient P has subscribed to, and based on that the configuration of the firmware may be adapted such that such features are enabled for the patient which are included in the subscription.

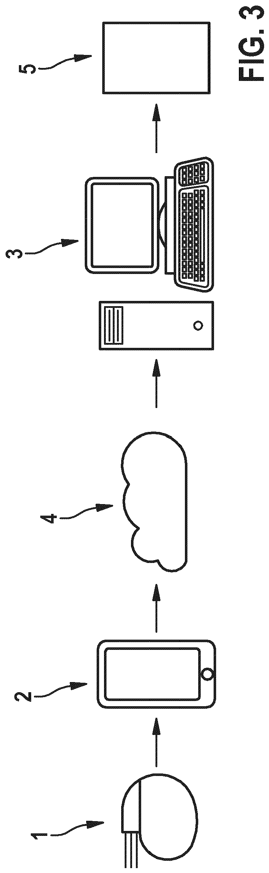

Alternatively or in addition, the remote management system 3 may check if the patient P has fulfilled his/her obligations under the subscription which he/she has subscribed to. For example, at the remote management assistant 3 it may be checked whether the patient P has made the payments required under the subscription or exercised in accordance with a regimen prescribed by the clinician. If this is not the case, features of the medical device 1, 1', 1" may be disabled such that the features of the medical device 1, 1', 1" are no longer available to the patient P. For this, as illustrated schematically in Fig. 3, the remote management system 3 may for example revert to a payment or subscription record 5 associated with the particular patient indicative whether payments and/or other obligations under the subscription have been fulfilled by the patient.

In order to ensure that only such features are available to the patient which should be available to the patient under the current subscription, it may be required that the medical device 1, 1', 1" regularly checks in with the remote management system 3. Such checking-in may require the establishing of a communication in between the remote management system 3 and the medical device 1, 1', 1". If, for a prolonged period of time, it is found that no communication is established between the medical device 1, 1', 1" and the remote management system 3, the medical device 1, 1', 1" may by itself adapt the configuration of the firmware such that certain features are disabled and are no longer available for the patient.

In particular, the medical device 1, 1', 1" may check whether a time period longer than a predefined timeout period has elapsed since a prior communication to the remote management system 3. If this is the case, the medical device 1, 1', 1" may disable certain features. In this way it can be ensured that a patient does not terminate a subscription and, nevertheless, continues to have access to features of the medical device 1, 1', 1" under the subscription which has recently been terminated by the patient.

Features which may be selectively enabled or disabled or modified based on a subscription may for example include (also dependent on the type of medical device):
- the displaying of diagnostic information e.g. on the patient device 2: the displaying of diagnostic information on the patient device 2 (in case the medical device for example is an implantable medical device 1) may be enabled or disabled or may be modified such that for example enhanced graphical information (under a higher subscription level) or only basic literal information (under a lower subscription level) are displayed;
- a diagnostic resolution: under a higher subscription level for example a recording of electrocardiogram signals with a higher resolution then under a lower subscription level may be offered;
- detection features: an atrial fibrillation detection feature, a syncope detection feature or an ectopy detection feature, for example, in a cardiac device may be enabled or disabled dependent on the subscription level;
- pain management features (in a pain management device): options for a user control may be enhanced or decreased, for example relating to the dynamics of a device response, dependent on the subscription level in a pain management device.

Other features of a firmware which may be selectively enabled, disabled or modified in a medica device 1, 1', 1" are conceivable.

### List of reference numerals

- 1, 1', 1": Medical device
- 11: Electrode lead
- 12: Electrode lead
- 13: Electrode lead
- 14: Processing circuitry
- 2: Patient device
- 3: Remote management system
- 4: Public communication network
- 5: Payment or subscription record
- P: Patient
- H: Patient's heart

## Claims

1. A system for managing operation of a medical device (1, 1', 1"), the system comprising:
a medical device (1, 1', 1") configured to carry out therapeutic and/or diagnostic functions in or on a patient (P), wherein the medical device (1, 1', 1") comprises a configurable firmware for controlling operation of the medical device (1, 1', 1") for carrying out said therapeutic and/or diagnostic functions, and
a remote management system (3),
wherein the remote management system (3) is configured to effect an adaption of a configuration of the configurable firmware of the medical device (1, 1', 1") based on information relating to a subscription by which the patient (P) has subscribed to a medical service provided by operation of the medical device (1, 1', 1").

2. The system according to claim 1, wherein the medical device (1, 1', 1") is an implantable medical device configured for implantation in a patient (P).

3. The system according to claim 2, wherein the medical device (1, 1', 1") is a implantable cardiac stimulation device or an implantable cardiac monitoring device or an implantable neuro-stimulation device.

4. The system according to claim 1, wherein the medical device (1, 1', 1") is a wearable medical device configured to be worn by a patient (P).

5. The system according to claim 1, wherein the medical device (1, 1', 1") is a medical point-of-care device configured to operate externally to the patient (P).

6. The system according to one of the preceding claims, wherein the remote management system (3) is configured to communicate with a patient device (2) resting outside of the patient (P), wherein the patient device (2) is configured to communicate with the medical device (1, 1', 1").

7. The system according to one of the preceding claims, wherein the firmware is programmed to include a complete set of features for carrying out said therapeutic and/or diagnostic functions, wherein the firmware is configurable to enable a subset of features of said complete set of features and disable features of said complete set of features not included in the subset based on said information relating to a subscription by which the patient (P) has subscribed to a medical service provided by operation of the medical device (1, 1', 1").

8. The system according to one of the preceding claims, wherein the remote management system (3) is configured, for effecting said adaption of the configuration, to check a subscription level of the patient (P) and/or to check whether the patient (P) has fulfilled the patient's obligations associated with a subscription level.

9. The system according to one of the preceding claims, wherein the remote management system (3) is configured to adapt the configuration of the firmware to enable a first set of features for providing a medical service in case the patient (P) has subscribed to a first subscription level and to adapt the configuration of the firmware to enable a second set of features for providing a medical service in case the patient (P) has subscribed to a second subscription level.

10. The system according to one of the preceding claims, wherein the remote management system (3) is configured, by effecting said adaption of the configuration of the configurable firmware of the medical device (1, 1', 1"), to expand or limit at least one feature for providing a medical service.

11. The system according to claim 10, wherein the expanding of at least one feature includes: enabling a predefined function to be carried out by the medical device (1, 1', 1") or increasing a capability of a predefined function to be carried out by the medical device (1, 1', 1").

12. The system according to claim 10 or 11, wherein the limiting of at least one feature includes: disabling a predefined function such that it is not carried out by the medical device (1, 1', 1") or decreasing a capability of a predefined function to be carried out by the medical device (1, 1', 1").

13. The system according to one of the preceding claims, wherein the medical device (1, 1', 1") is configured to cause an adaption of the configuration of the firmware based on a pre-defined timeout period following a prior communication with the remote management system (3).

14. The system according to claim 13, wherein the medical device (1, 1', 1") is configured to cause an adaption of the configuration of the firmware to limit at least one feature for providing a medical service in case the predefined timeout period following the prior communication with the remote management system (3) is exceeded without another communication with the remote management system (3) being established within the timeout period.

15. A method for operating a system for managing operation of a medical device (1, 1', 1"), the method comprising:
providing a medical device (1, 1', 1") configured to carry out therapeutic and/or diagnostic functions in or on a patient (P), wherein the medical device (1, 1', 1") comprises a configurable firmware for controlling operation of the medical device (1, 1', 1") for carrying out said therapeutic and/or diagnostic functions;
providing a remote management system (3); and
effecting, by remote management system (3), an adaption of a configuration of the configurable firmware of the medical device (1, 1', 1") based on information relating to a subscription by which the patient (P) has subscribed to a medical service provided by operation of the medical device (1, 1', 1").
